# EUROPÄISCHE PATENTSCHRIFT

(11) **EP 4 385 430 B1**
(45) Veröffentlichungstag und Bekanntmachung des Hinweises auf die Patenterteilung: **03.09.2025**
(21) Anmeldenummer: 23217016.7
(22) Anmeldetag: 15.12.2023
(51) Int. Cl.: A61B 17/22, A61B 17/32, B06B 1/06, A61B 17/00

(54) **HALTEVORRICHTUNG FÜR EINE LITHOTRIPSIEVORRICHTUNG UND LITHOTRIPSIEVORRICHTUNG ZUM ZERTRÜMMERN VON KÖRPERSTEINEN**
HOLDING DEVICE FOR A LITHOTRIPSY DEVICE AND LITHOTRIPSY DEVICE FOR CRUSHING BODY STONES
DISPOSITIF DE MAINTIEN POUR UN DISPOSITIF DE LITHOTRIPSIE ET DISPOSITIF DE LITHOTRIPSIE DESTINÉ À LA DESTRUCTION DE CALCULS CORPORELS

(30) Priorität: 15.12.2022 DE 102022133521
(43) Veröffentlichungstag der Anmeldung: 19.06.2024
(73) Patentinhaber: Karl Storz SE & Co. KG, 78532 Tuttlingen (DE)
(72) Erfinder: GLÖGGLER, Bernhard, 78532 Tuttlingen (DE); KRATTIGER, Beat, 78532 Tuttlingen (DE)

(56) Entgegenhaltungen:
- DE-A1- 102022 109 138
- US-A- 5 397 293
- US-A1- 2002 010 486
- US-B2- 11 357 523

## Beschreibung

Die Erfindung betrifft eine Haltevorrichtung für eine Lithotripsievorrichtung zum Zertrümmern von Körpersteinen, wobei die Haltevorrichtung ein Gehäuse mit einem distalen Ende und einem proximalen Ende aufweist und an dem distalen Ende eine Sonotrode verbindbar ist, wobei in dem Gehäuse ein Beschleunigungsrohr mit einer Längsmittelachse, einem Hohlraum, einem proximalen Ende, einem distalen Ende und mit einem bewegbaren Projektil innerhalb des Hohlraums zur Stoßanregung der Sonotrode, ein proximalseitiges Anschlagselement am proximalen Ende und ein distalseitiges Anschlagselement am distalen Ende des Beschleunigungsrohrs angeordnet sind, und der Haltevorrichtung eine Krafterzeugungseinrichtung zum Erzeugen einer Kraft zum Hin- und/oder Zurückbewegen des Projektils zwischen dem proximalseitigen Anschlagselement und dem distalseitigen Anschlagselement zuordenbar ist, und in dem Gehäuse eine Schwingungsanregungseinrichtung zur Schwingungsanregung der Sonotrode angeordnet ist. Des Weiteren betrifft die Erfindung eine Lithotripsievorrichtung, insbesondere intrakorporale Lithotripsievorrichtung, zum Zertrümmern von Körpersteinen.

Die Lithotripsie ist ein bekanntes Verfahren zum Zertrümmern von Körpersteinen, welche sich z.B. durch Kondensation und/oder Auskristallisation von Salzen und Eiweißen als sogenanntes Konkrement in Körperorganen, wie beispielsweise in der Blase oder Niere, bilden.

Wenn die Körpersteine zu groß für einen natürlichen Abgang sind und Beschwerden verursachen, müssen diese mit einem Lithotripter zerkleinert werden, sodass die zerkleinerten Steine durch natürliche Ausscheidung und/oder mittels einer Saug-Spül-Pumpe entfernt werden können. Die zu zertrümmernden Körpersteine sind häufig inhomogen mit unterschiedlichen Bestandteilen und/oder Festigkeiten aufgebaut.

Um die Steinzertrümmerungsleistung zu verbessern, werden vor allem in der intrakorporalen Lithotripsie Kombinationssysteme eingesetzt, welche zwei verschiedene Anregungs- und/oder Schwingungsquellen kombiniert verwenden. Dazu wird häufig zusätzlich zur konstanten Ultraschallenergie eine intermittierende, ballistische Schockwellenenergie zugeführt. Dies kann beispielsweise mittels eines ballistischen Antriebs mit Elektromagneten erfolgen, bei dem ein Prallkörper mittels der Elektromagneten beschleunigt wird und auf ein Horn und/oder den Sonotrodenkopf aufschlägt. Nachteilig hierbei ist, dass aufgrund der Erwärmung der Elektromagneten im Dauerbetrieb zumindest das distale Ende des Handgriffes eines solchen Lithotripters aktiv gekühlt werden muss. Zudem ist das Instrument durch das für die Kühlung notwendige, fest verbundene Hybridkabel schwer und ergonomisch unhandlich.

Des Weiteren ist es bekannt, eine oszillierende Masse in Ringform um eine Sonotrode herum anzuordnen und mittels einer Feder an einem axialen Anschlag der Sonotrode anzupressen. Hierbei beschleunigt die Ultraschallschwingung die Masse vom Anschlag weg, wodurch die Feder komprimiert wird und die Masse wieder auf den Anschlag hin beschleunigt. Nachteilig ist bei diesem in Längsrichtung der Sonotrode wirkenden Feder-Masse-System, dass dieses nur eine begrenzte Schlagkraft erlaubt. Des Weiteren ist die ballistische Funktion nicht beliebig zuschaltbar und in ihrer Stärke, Wiederholfrequenz und/oder Kadenz nicht einstellbar.

Bei ballistischen Systemen mit einem reinen pneumatischen Antrieb oder Kombinationssystemen mit Pneumatikeinheit wird das Projektil in einem Beschleunigungsrohr in distaler Richtung bewegt und muss nach einem Schlag auf die Sonde oder Sonotrode in proximaler Richtung zurückbewegt werden. Für diese Rückstellung des Projektils kann distalseitig ein Luftreservoir mit Ventil angeordnet sein oder, wie in der DE 20 2014 007 692 U1 beschrieben, ist eine Staukammer um das Beschleunigungsrohr angeordnet. Nachteilig bei beidem Rückstellungsvarianten ist, dass aufgrund des Luftreservoirs oder der Staukammer der Bauraum in der Pneumatikeinheit und/oder Lithotripsievorrichtung eingeschränkt und die Durchführung von anderen Komponenten, wie beispielsweise Spül- und Absaugleitungen, konstruktiv erschwert ist.

Bei der Schwingungsanregung mittels Ultraschall ist es zudem bekannt, in Ultraschallwandlern einen Ultraschall-Schwingungskompensator auf der Gegenseite des Horns und somit am schwingenden proximalen Ende des Ultraschallkonverters anzuordnen, welcher als mechanisches Befestigungselement zwischen einem ruhenden Gehäuse der Lithotripsievorrichtung und dem schwingenden, proximalen Ende des Ultraschallkonverters dient. Bei gezielter Auslegung dieses Ultraschall-Schwingungskompensators reduziert dieser die Ultraschall-schwingungen über seine Länge auf ein Minimum oder Null, ohne dass dabei der Ultraschallkonverter merklich in seiner Resonanzfrequenz verstimmt wird. Die Abmessungen eines solchen Ultraschall-Schwingungskompensators sind jedoch nicht beliebig ausbildbar, da ansonsten eine unerwünschte Verstimmung des Ultraschallkonverters erfolgt, unerwünschte Querschwingungen angeregt werden und/oder unangenehme Geräusche auftreten können. Zudem kann die Gehäuselänge in proximaler Richtung nicht frei gestaltet werden.

Aus der US 2002/0010486 A1 ist ein intrakorporaler Lithotripter bekannt. Er umfasst sowohl eine von einem elektrisch angesteuerten Piezo-Ultraschallwandler angeregte hohle Metallsonde als auch eine von einem reversibel angetriebenen Schlagteil angeregte Stoßsonde. Während der Operation kann zwischen den beiden Steinzertrümmerungsmöglichkeiten umgeschaltet werden.

Die Offenlegungsschrift DE 10 2022 109 138 A1 lehrt eine Lithotripsievorrichtung, deren Sonotrode von einem in einem Führungsrohr hin- und herbeweglichen Projektil angeregt wird. Das Projektil wird mittels einer im Führungsrohr befindlichen Steuerhülse bei kontinuierlichem Zu- und/oder Abführen eines Druckmediums selbstanregend in andauernder Bewegung gehalten.

Die Patentschrift US 11,357,523 A1 betrifft eine Lithotripsievorrichtung, deren Sonde von einer ersten Antriebseinrichtung periodisch und von einer zweiten Antriebseinrichtung gepulst ausgelenkt wird.

Das Patent US 5,397,293 A offenbart eine Ultraschall-Angioplastie-Vorrichtung mit einem Ultraschallgenerator und einem ummantelten Katheter-Draht. Die Ummantelung dämpft nur die Querschwingungen des Katheter-Drahts, nicht aber dessen Aaxialbewegung.

Aufgabe der Erfindung ist es, den Stand der Technik zu verbessern.

Gelöst wird die Aufgabe durch eine Haltevorrichtung für eine Lithotripsievorrichtung zum Zertrümmern von Körpersteinen, wobei die Haltevorrichtung ein Gehäuse mit einem distalen Ende und einem proximalen Ende aufweist und an dem distalen Ende eine Sonotrode verbindbar ist, wobei in dem Gehäuse ein Beschleunigungsrohr mit einer Längsmittelachse, einem Hohlraum, einem proximalen Ende, einem distalen Ende und mit einem bewegbaren Projektil innerhalb des Hohlraums zur Stoßanregung der Sonotrode, ein proximalseitiges Anschlagselement am proximalen Ende und ein distalseitiges Anschlagselement am distalen Ende des Beschleunigungsrohrs angeordnet sind, und der Haltevorrichtung eine Krafterzeugungseinrichtung zum Erzeugen einer Kraft zum Hin- und/oder Zurückbewegen des Projektils zwischen dem proximalseitigen Anschlagselement und dem distalseitigen Anschlagselement zuordenbar ist, und in dem Gehäuse eine Schwingungsanregungseinrichtung zur Schwingungsanregung der Sonotrode angeordnet ist, wobei die Haltevorrichtung eine Schwingungskompensationseinrichtung mit mindestens einer Masse und mindestens einem Federelement aufweist, sodass mittels der Schwingungskompensationseinrichtung das Beschleunigungsrohr von der Schwingungsanregung mittels der Schwingungsanregungseinrichtung entkoppelbar ist.

Somit wird ein Handstück für eine Lithotripsievorrichtung mit einer kombinierten Schlag- und Schwingungsanregung und einer Schwingungskompensationseinrichtung für die Kompensation von Ultraschallschwingungen bereitgestellt. Folglich werden unerwünschte angeregte Schwingungen erzeugt von der Schwingungsanregungseinrichtung auf das Beschleunigungsrohr verhindert oder zumindest vermindert, wodurch die Schwingungsanregungseinrichtung und der ballistische Antrieb mittels der Krafterzeugungseinrichtung unabhängig voneinander einstellbar und betreibbar sind. Es ist besonders vorteilhaft, dass die Haltevorrichtung für eine kombinierte Lithotripsievorrichtung aufgrund der multifunktionalen Schwingungskompensationseinrichtung ein geringes Gewicht aufweist, nicht gekühlt werden muss und eine beliebig zuschaltbare und einstellbare Schlaganregung aufweist. Somit ist das ruhende und somit stationäre Beschleunigungsrohr in dem Gehäuse der Haltevorrichtung sicher gehalten und mittels der Schwingungskompensationseinrichtung gleichzeitig zuverlässig von den starken Ultraschallschwingungen am distalen Ende des Ultraschallkonverters entkoppelt.

Dadurch, dass die Schwingungskompensationseinrichtung mindestens eine Masse als eigene und unabhängige Ruhemasse aufweist, sind die Schwingungskompensationseinrichtung und das Gehäuse unabhängig voneinander ausgestaltbar. Dabei ist die Schwingungskompensationseinrichtung für die jeweiligen Ultraschall-Schwingungen, beispielsweise mit einer Frequenz von circa 27 kHz, exakt abgestimmt und aufgrund der freien Ausgestaltbarkeit der Schwingungskompensationseinrichtung weist diese eine λ/4-Geometrie auf, welche der Resonanzfrequenz des Ultraschallkonverters entspricht und diesen dadurch nicht oder kaum verstimmt.

Aufgrund der multifunktionalen Schwingungskompensationseinrichtung ist eine leichte Haltevorrichtung als Handstück einer kombinierten Lithotripsievorrichtung mit optimal nutzbarem Bauraum bereitgestellt, welche konstruktiv einfach und kostengünstig herstellbar ist.

Ein wesentlicher Gedanke der Erfindung beruht darauf, mittels einer Schwingungskompensationseinrichtung mit mindestens einem Federelement und mit mindestens einer Masse als eigene, integrierte Ruhemasse eine freie Ausbildung dieser Schwingungskompensationseinrichtung unabhängig von der Ausgestaltung des Gehäuses innerhalb der Haltevorrichtung zu gewährleisten und dadurch neben der Schwingungsentkopplung des Beschleunigungsrohres der ballistischen Schlaganregung gleichzeitig mittels der Schwingungskompensationseinrichtung weitere vorteilhafte Funktionen für eine kombinierte Lithotripsievorrichtung bereitzustellen und dabei einen kompakten Bauraum innerhalb der Haltevorrichtung effizient auszunutzen.

Folgendes Begriffliche sei erläutert:
Bei einer "Lithotripsievorrichtung" (auch "Lithotripter" genannt) handelt es sich insbesondere um eine Vorrichtung zum Zertrümmern von Körpersteinen durch Stöße, Stoßwellen und/oder Verformungswellen. Unter einer Lithotripsievorrichtung werden insbesondere verschiedene Bestandteile, Bau- und/oder Funktionskomponenten eines Lithotripters verstanden. Die Lithotripsievorrichtung kann einen Lithotripter vollständig oder teilweise ausbilden. Bei einer Lithotripsievorrichtung kann es sich insbesondere um eine intrakorporale oder extrakorporale Lithotripsievorrichtung handeln. Im Falle einer intrakorporalen Lithotripsievorrichtung kann diese zusätzlich eine Spül-/Saugpumpe aufweisen. Die Lithotripsievorrichtung kann als Handgerät ausgebildet sein und/oder ein Endoskop aufweisen oder in ein Endoskop eingeschoben werden. Die Lithotripsievorrichtung ist insbesondere autoklavierbar und weist beispielsweise Instrumentenstahl und/oder Kunststoff auf. Die Lithotripsievorrichtung kann weitere Komponenten, wie ein Steuer- und/oder Versorgungsgerät aufweisen oder diese sind der Lithotripsievorrichtung zugeordnet. Eine Lithotripsievorrichtung ist insbesondere eine kombinierte Lithotripsievorrichtung mit einer ballistischen und/oder pneumatischen Einheit und zuordenbaren Krafterzeugungseinrichtung und einer Schwingungsanregungseinrichtung. Mittels der ballistischen und/oder pneumatischen Einheit und der zugeordneten Krafterzeugungseinrichtung wird mittels einer Stoßenergie beim Anschlagen eines Projektils an einem distalseitigen Anschlagselement insbesondere der Sonotrode direkt oder indirekt eine gezielt geformte Verformungswelle aufgeprägt. Die Verformungswelle bewirkt insbesondere eine translatorische Bewegung der Sonotrode, welche aufgrund der Auslenkung eine Steinzertrümmerung bewirkt. Gleichzeitig wird bei der kombinierten Lithotripsievorrichtung neben dem mechanischen Stoß die Sonotrode zusätzlich insbesondere mittels einer Schwingungsanregungseinrichtung, beispielsweise mit einem Ultraschallwandler, in eine Schwingung, insbesondere longitudinale Schwingung und/oder Querschwingung, angeregt. Somit ist die Sonotrode insbesondere als Wellenleiter für die Schwingungswellen erzeugt von der Schwingungsanregungseinrichtung und für die Verformungswellen des Projektils ausgebildet.

Unter "Körpersteinen" (auch "Konkrement" genannt) werden insbesondere alle Steine in einem menschlichen oder tierischen Körper verstanden, welche sich z.B. aus Salzen und Eiweißen durch Kristallisation und/oder Kondensation bilden. Bei Körpersteinen kann es sich beispielsweise um Gallensteine, Harnsteine, Nierensteine und/oder Speichelsteine handeln. Durch Einwirken der Sonotrode und/oder Hohlsonde auf den Körperstein entstehen insbesondere Körpersteinkerne (auch Bohrkerne genannt) und/oder Körpersteinfragmente.

Eine "Haltevorrichtung" (auch "Handstück" genannt) ist insbesondere ein Hand- und/oder Halteteil der Lithotripsievorrichtung. Bei der Haltevorrichtung kann es sich insbesondere um eine Handhabe zur manuellen und/oder automatisierten Bedienung und/oder Verbindung der Lithotripsievorrichtung handeln. Eine Haltevorrichtung kann auch an einem distalen Ende eines Roboterarms angeordnet, verbunden und/oder automatisiert geführt sein. Die Haltevorrichtung weist insbesondere ein Gehäuse auf. Die Haltevorrichtung kann auch zwei- oder mehrteilig ausgebildet sein. Beispielsweise kann die Haltevorrichtung ein separates Gehäuse für eine Pneumatikeinheit und ein separates Gehäuse für die Schwingungsanregungseinrichtung aufweisen.

Unter "distalseitig" und "distal" wird eine patientenkörpernahe und somit benutzerferne Anordnung und/oder ein entsprechendes Ende oder Abschnitt verstanden. Dementsprechend wird unter "proximalseitig" oder "proximal" eine benutzernahe und somit patientenkörperferne Anordnung oder ein entsprechendes Ende oder Abschnitt verstanden.

Eine "Sonotrode" ist insbesondere ein Bauteil, welches durch das Einwirken und/oder Einleiten von mechanischen Schwingungen selbst in Schwingung und/oder Resonanzschwingung versetzt wird. Die Sonotrode ist insbesondere als Wellenleiter für die Schwingungswellen erzeugt von der Schwingungsanregungseinrichtung und für die Verformungswellen durch Anschlag des mittels der Krafterzeugungseinrichtung beschleunigten Projektils ausgebildet. Die Sonotrode ist insbesondere mit der Schwingungsanregungseinrichtung, dem Ultraschallwandler und/oder dem Horn direkt oder indirekt verbunden. Beispielsweise ist die Sonotrode in das distalseitige Ende des Horns eingeschraubt. Die Sonotrode weist insbesondere an ihrem proximalen Ende einen Sonotrodenkopf zum Aufnehmen, Weiterleiten und/oder Fokussieren von Ultraschallwellen und an ihrem distalen Ende eine Sonotrodenspitze zum unmittelbaren und/oder mittelbaren Beaufschlagen und/oder Kontaktieren von Körpersteinen auf. Die Sonotrode ist insbesondere derart geformt, dass diese optimal die Schwingungswellen, die Ultraschallschwingung und/die Verformungswellen an ihrem distalen Ende in den Körper, die zu behandelnde Körperregion und/oder direkt auf den zu zertrümmernden Körperstein einleitet. Im Falle der Ultraschallanregung arbeitet die Sonotrode insbesondere im Ultraschallbereich mit einem Frequenzbereich von 20 kHz bis 90 kHz, bevorzugt von 20 kHz bis 34 kHz. Die Sonotrode weist insbesondere Stahl, Titan, Aluminium und/oder Carbon auf. Bei einer Sonotrode handelt es sich insbesondere um eine Sonde, welche beispielsweise stab-, röhren- und/oder schlauchförmige ausgebildet ist. Die Sonotrode kann einstückig oder mehrteilig ausgebildet sein. Die Sonotrode weist insbesondere einen Durchmesser in einem Bereich von 0,5 mm bis 4,5 mm, insbesondere von 0,8 mm bis 3,8 mm, auf.

Ein "Beschleunigungsrohr" ist insbesondere ein länglicher Hohlkörper, dessen Länge eine größere Abmessung aufweist als sein Durchmesser. Das Beschleunigungsrohr weist in seinem Inneren insbesondere einen Hohlraum auf, in dem ein Projektil sich frei in Längsrichtung bewegen kann. Des Weiteren weist das Beschleunigungsrohr insbesondere ein proximales Ende und ein distales Ende auf, welche räumlich, nach Abzug der Projektillänge, ungefähr die maximale Beschleunigungsstrecke festlegen. Das Beschleunigungsrohr ist distalseitig und/oder an seinem distalen Endabschnitt insbesondere zumindest teilweise von dem Horn und einem mit dem Horn verbundenen oder zugeordneten Bolzen umgeben. Bei einer pneumatischen Krafterzeugungseinrichtung weist das Beschleunigungsrohr zumindest eine Öffnung zum Eintritt und/oder Austritt eines Druckmediums, insbesondere Druckluft, auf. Das Beschleunigungsrohr weist als Material insbesondere ein Metall auf.

Ein "Anschlagselement" ist insbesondere ein gewollter Endpunkt der Bewegung des Projektils entlang der Beschleunigungsstrecke innerhalb des Hohlraums des Beschleunigungsrohres, an dem das beschleunigte Projektil gegen das Anschlagselement schlägt, abgebremst und/oder in die Gegenrichtung bewegt wird. Ein distalseitiges Anschlagselement ist insbesondere am und/oder im distalen Ende des Beschleunigungsrohres und/oder innerhalb des Hohlraums in einem Bereich des distalen Abschnittes des Beschleunigungsrohres angeordnet. Das distalseitige Anschlagselement überträgt insbesondere direkt oder indirekt den Stoß des Projektils auf die Sonotrode. Bei dem distalseitigen Anschlagselement kann es sich beispielsweise um eine proximalseitige Wand des Horns, ein Federelement oder um eine proximalseitige Wand eines Halters eines Federelementes handeln. Das proximalseitige Anschlagselement ist insbesondere am und/oder im proximalen Ende des Beschleunigungsrohrs oder innerhalb des Hohlraums in einem proximalen Abschnitt des Beschleunigungsrohrs angeordnet. Bei dem proximalseitigen Anschlagselement kann es sich beispielsweise um eine Wand des Gehäuses und/oder um ein Federelement handeln.

Ein "Projektil" ist insbesondere ein Körper, welcher innerhalb des Hohlraums des Beschleunigungsrohres frei entlang der Beschleunigungsstrecke beweglich ist. Das Projektil ist insbesondere zwischen dem proximalseitigen Anschlagselement und dem distalseitigen Anschlagselement innerhalb des dazwischen angeordneten Hohlraums des Beschleunigungsrohres hin- und zurückbewegbar. Prinzipiell kann das Projektil jegliche Form aufweisen. Beispielsweise kann das Projektil die Form eines Bolzens oder einer Kugel aufweisen. Das Projektil weist insbesondere harten Stahl und/oder schwachmagnetische Eigenschaften auf. Für die freie Beweglichkeit weist das Projektil insbesondere einen etwas geringeren Außendurchmesser als der Durchmesser des Hohlraumes des Beschleunigungsrohres auf. Beispielsweise kann das Projektil einen Außendurchmesser von 8 mm, insbesondere 6 mm, oder 4 mm aufweisen.

Das Projektil kann insbesondere entlang der Beschleunigungsstrecke beständig, sich wiederholend oder einzelausgelöst mittels der Krafterzeugungseinrichtung hin- und/oder herbewegt werden. Bevorzugt wird das Projektil intermittierend und/oder oszillierend zwischen dem proximalseitigen Anschlagselement und dem distalseitigen Anschlagselement hin und her bewegt.

Bei einer "Krafterzeugungseinrichtung" kann es sich prinzipiell um jegliche Art von Einrichtung handeln, welche eine Kraft auf das Projektil und somit eine Bewegung des Projektils bewirkt. Bei der Krafterzeugungseinrichtung kann es sich beispielsweise um eine Vorrichtung handeln, welche mittels Lasers, eines Druckmediums, beispielsweise pneumatisch mithilfe von Druckluft, mittels eines elektromagnetischen Feldes und/oder mittels einer mechanischen Vorrichtung das Projektil beschleunigt. Eine pneumatisch Krafterzeugungseinrichtung kann insbesondere mittels eines Zuführens und/oder Abführens eines Druckmediums eine lineare Bewegung des Projektils im Hohlraum des Beschleunigungsrohres bewirken. Das Druckmedium strömt insbesondere durch mindestens eine proximalseitige Öffnung des Beschleunigungsrohres in den Hohlraum des Beschleunigungsrohres und drückt und beschleunigt das Projektil in distale Richtung.

Eine "Schwingungsanregungseinrichtung" ist insbesondere jegliche Einrichtung zum Erzeugen von Schwingungen im Ultraschallbereich. Die Schwingungsanregungseinrichtung weist insbesondere einen Ultraschallwandler (auch Ultraschallkonverter genannt) auf, welcher eine zugeführte Wechselspannung mit einer bestimmten Frequenz in eine mechanische Schwingungsfrequenz umsetzt, oder die Schwingungsanregungseinrichtung ist durch den Ultraschallwandler ausgebildet. Der Ultraschallwandler ist insbesondere ein elektromechanischer Wandler unter Ausnutzung des piezoelektrischen Effektes. Durch Anlegen der von einem Ultraschallgenerator erzeugten elektrischen Wechselspannung wird eine mechanische Schwingung aufgrund eines Verformens des Ultraschallwandlers erzeugt. Der Ultraschallwandler weist insbesondere ein Piezoelement oder mehrere, bevorzugt gestapelte, Piezoelemente auf. Bevorzugt weist der Ultraschallwandler mindestens zwei Piezoelemente auf, wobei zwischen den Piezoelementen ein elektrischer Leiter, beispielsweise eine Kupferscheibe, angeordnet ist. Ein distalseitiges Piezoelement des Ultraschallwandlers liegt insbesondere direkt an einer proximalen Wand eines Hornes an. Proximalseitig des Piezoelementes oder der Piezoelemente ist insbesondere ein Gegenlager angeordnet. Zwischen dem proximalen Ende des proximalseitigen Piezoelementes und dem distalen Ende des Gegenlagers kann eine Zwischenscheibe angeordnet sein. Das Piezoelement, die Piezoelemente, die Zwischenscheibe und/oder das Gegenlager können insbesondere um einen Bolzen, insbesondere Hohlbolzen, angeordnet sein, welcher proximalseitig des Horns angeordnet ist.

Ein "Horn" ist insbesondere ein Bauteil, welches zwischen dem Ultraschallwandler und/oder einem Piezoelement und der Sonotrode angeordnet ist. Das Horn dient insbesondere dazu, die vom Ultraschallwandler erzeugten Ultraschallwellen zur Sonotrode zu übertragen, weiterzuleiten, zu fokussieren und/oder auszurichten. Dazu kann das Horn sich in einer Übertragungsrichtung verjüngen und direkt oder indirekt die Ultraschallwellen auf einen Sondenkopf übertragen. Durch eine Querschnittsverminderung des Horns in Übertragungsrichtung wird insbesondere eine Amplitudenvergrößerung erzielt. Das Horn kann auch zur Befestigung der Sonotrode verwendet werden. Gleichzeitig dient das Horn insbesondere zusammen mit einem Gegenlager und/oder einer Zwischenscheibe zur beidseitigen mechanischen Halterung des Piezoelementes oder der Piezoelemente. Entgegen der Übertragungsrichtung, insbesondere proximalseitig, schließt das Horn mit einer Wand ab. Proximalseitig dieser Wand ist insbesondere ein Bolzen angeordnet. Bei dem Bolzen handelt es sich bevorzugt um einen Hohlbolzen. Das Horn und der Bolzen können insbesondere als zwei separate Bauteile ausgebildet sein. Bevorzugt handelt es sich bei dem Horn und dem Bolzen um ein einstückiges Bauteil, wobei ein Hornabschnitt dem konventionellen Horn entspricht und entgegen der Übertragungsrichtung, insbesondere in proximaler Richtung, insbesondere gestuft in den Hohlbolzenabschnitt mit einem geringeren Querschnitt übergeht. Um den Hohlbolzenabschnitt ist mindestens ein Piezoelement mit elektrischem Kontakt und das Gegenlager und/oder zusätzlich eine dazwischen angeordnete Zwischenscheibe zwischen dem proximalseitigen Piezoelement und der distalen Seite des Gegenlagers angeordnet. Das Gegenlager ist insbesondere auf dem Hohlbolzen oder dem Hohlbolzenabschnitt aufgeschraubt und spannt dadurch zumindest ein Piezoelement und/oder die Zwischenscheibe ein. Das Gegenlager kann als Schraubenmutter ausgebildet sein. Ein proximaler Endabschnitt des Hohlbolzenabschnittes und/oder des Hohlbolzens steht insbesondere in proximaler Richtung über das proximale Ende des Gegenlagers hinaus. Über diesen hinausragenden proximalen Endabschnitt des Hohlbolzenabschnittes und/oder Hohlbolzens ist insbesondere ein Verbindungsabschnitt der Schwingungskompensationseinrichtung umgebend angeordnet und/oder verbunden. Bevorzugt ist der Verbindungsabschnitt der Schwingungskompensationseinrichtung auf den proximalen Abschnitt des Hohlbolzenabschnittes und/oder Hohlbolzens aufgeschraubt und somit mit diesem mechanisch gekoppelt. Dadurch ist insbesondere das proximale Ende des Ultraschallwandlers mechanisch mit dem Verbindungsabschnitt der Schwingungskompensationseinrichtung gekoppelt.

Eine "Schwingungskompensationseinrichtung" (auch "Amplituden-Kompensator" genannt) ist insbesondere ein Bauteil oder eine Baugruppe, welche mindestens eine Masse und mindestens ein Federelement aufweist. Die Schwingungskompensationseinrichtung dient insbesondere zur schwingungstechnischen Entkopplung des Beschleunigungsrohres des ballistischen und/oder pneumatischen Antriebs von der Schwingungsanregung mittels der Schwingungsanregungseinrichtung. Das Federelement ist insbesondere auf der distalen Seite und die Masse als Ruhemasse auf der proximalen Seite der Schwingungskompensationseinrichtung angeordnet. Die Schwingungskompensationseinrichtung weist insbesondere einen durchgehenden Hohlraum in ihrer Masse und ihrem Federelement auf, durch welchen das Beschleunigungsrohr durchführbar ist, sodass das Beschleunigungsrohr an seiner Außenoberfläche von der Schwingungskompensationseinrichtung umgeben ist.

Die Schwingungskompensationseinrichtung weist als weitere Bauteile insbesondere mindestens ein Verbindungselement zum Verbinden der Masse und mindestens ein Dichtungselement, wie beispielsweise einen O-Ring auf. Das Dichtungselement wirkt gleichzeitig als Dämpfungselement. Die Schwingungskompensationseinrichtung kann auch mehrere, beispielsweise parallel zueinander angeordnete Federelemente und/oder mehrere Massen aufweisen.

Ein "Federelement" ist insbesondere ein Bauteil und/oder ein Abschnitt der Schwingungskompensationseinrichtung, welches sich ausreichend elastisch verformen lässt. Das Federelement weist insbesondere Metall und/oder Kunststoff auf. Bei einem Federelement kann es sich insbesondere um eine konventionelle Feder, wie beispielsweise eine Schraubenfeder und somit einen in Schraubenform gewickelten Draht handeln. Bevorzugt handelt es sich bei dem Federelement um einen dünnwandigen Rohrabschnitt, welcher insbesondere als λ/4-Masse-Federelement wirkt. Die Masse und/oder die gesamte Schwingungskompensationseinrichtung weist insbesondere Aluminium und/oder Stahl auf. Bevorzugt weist der gesamte Amplituden-Kompensator Aluminium und/oder eine Aluminiumlegierung auf. Während im Betrieb das Federelement der Schwingungskompensationseinrichtung schwingt und dadurch dämpfend wirkt, bleibt die Masse aufgrund ihres deutlich größeren Gewichtes insbesondere in Ruhe und schwingt gerade nicht.

Ein "Amplitudenknoten" ist eine Stelle im Bereich einer stehenden Welle oder der Überlagerung von zwei gegenläufigen fortschreitenden Wellen gleicher Frequenz und gleicher Amplitude entstanden durch Reflektion, deren Auslenkung immer bei Null bleibt. Die "Wellenlänge" einer periodischen Welle ist insbesondere der kleinste Abstand zweier Punkte gleicher Phasen. Beispielsweise ist die Wellenlänge der Abstand zwischen zwei maximalen Amplituden.

Unter einer "λ/4-Geometrie" der Schwingungskompensationseinrichtung wird insbesondere verstanden, dass bei einer Befestigung des Ultraschallwandlers an einem Amplitudenbauch (Schwingungsbauch) das Federelement der Schwingungskompensationseinrichtung einen Abstand von λ/4 zur proximalen Masse der Schwingungskompensationseinrichtung, d.h. zu dem deren entsprechenden Amplitudenknoten aufweist. Dadurch liegt die maximale Amplitude an dem distalen Ende des Federelements vor, welche aufgrund der elastischen Eigenschaften von dem Federelement gegen proximal gedämpft wird, sodass in der proximalseitig angeordneten Masse der Schwingungskompensationseinrichtung nur noch eine geringe oder gar keine Ultraschallrestamplitude vorliegt, wodurch schwingungstechnisch eine Entkopplung zu dem ruhenden Beschleunigungsrohr erzielt wird. Dadurch, dass die Schwingungskompensationseinrichtung eine λ/4-Geometrie aufweist, entspricht diese der Resonanzfrequenz des Ultraschallwandlers und verstimmt den Ultraschallwandler nicht oder nur sehr geringfügig.

Eine "Längsmittelachse" ist insbesondere die Achse des jeweiligen Körpers oder Bauteiles, welche der Richtung seiner größten Ausdehnung und/oder Abmessung entspricht. Bei der Längsmittelachse kann es sich auch um die Symmetrieachse des jeweiligen Körpers und/oder Bauteiles handeln.

Eine "Längsrichtung" ist insbesondere die Richtung der längsten Ausdehnung eines Bauteils und/oder Körpers. Die Längsrichtung ist insbesondere die Richtung entlang der Längsmittelachse der Masse, der Sonotrode und/oder des Beschleunigungsrohrs.

In einer weiteren Ausführungsform der Haltevorrichtung ist die Masse in ihrer Längsrichtung und/oder an ihrem proximalen Ende frei von einer Verbindung mit dem Gehäuse angeordnet.

Somit ist die Masse der Schwingungskompensationseinrichtung als in Längsrichtung freie Ruhemasse ausgebildet, welche gerade nicht Teil des Gehäuses ist oder mit dem Gehäuse proximalseitig verbunden ist. Dadurch können weitere Funktionen in die Schwingungskompensationseinrichtung frei integriert werden. Vor allem ist die Baulänge des Gehäuses und/oder der Haltevorrichtung insbesondere in proximaler Richtung unabhängig von der Ultraschall-Funktion des Ultraschallwandlers. Dadurch kann die Baulänge des Gehäuses auf die Länge des Beschleunigungsrohres angepasst oder frei gewählt werden. Indem die Schwingungskompensationseinrichtung unabhängig von der Gehäuserückwand ausgelegt ist, da in der Schwingungskompensationseinrichtung die Masse als eine von der Gehäuserückwand unabhängige Ruhemasse integriert ist, können die ballistischen und/oder pneumatischen Eigenschaften und somit die Stoßanregung unabhängig von den Ultraschall-Eigenschaften und somit unabhängig von der Schwingungsanregungseinrichtung optimiert und eingestellt werden. Dadurch kann das Beschleunigungsrohr optimal in dem Gehäuse eingebaut werden, auch wenn dieses für eine effiziente Funktion üblicherweise eine bestimmte Länge aufweisen muss. Durch die Entkopplung mittels der Schwingungskompensationseinrichtung und der in der Schwingungskompensationseinrichtung selbst integrierten Ruhemasse kann das Gehäuse der Haltevorrichtung beliebig, insbesondere in proximaler Richtung, verlängert werden, ohne dass dabei die Ultraschall-Verhältnisse der Schwingungsanregungseinrichtung sich ändern.

Um Quermomente der mittels der Schwingungsanregungseinrichtung erzeugten Schwingungen gezielt abzuleiten, ist die Masse im Wesentlichen quer zu ihrer Längsrichtung mittels mindestens eines Verbindungselementes direkt oder indirekt mit dem Gehäuse verbunden.

Dadurch werden Quermomente auf dem am Knotenpunkt des Horns elastisch und beweglich gelagerten Ultraschallkonverter mittels der Schwingungskompensationseinrichtung abgefangen und gezielt im Wesentlichen in Richtung quer zur Längsrichtung der Masse und/oder des Beschleunigungsrohres abgeleitet. Somit kann der Amplituden-Kompensator an das schwingende proximale Ende des Ultraschallwandlers und/oder den Hohlbolzen angeschraubt sein, während das dickere gegenüberliegende Bauteilende des Amplituden-Kompensators als Ruhemasse über seine Mantelfläche mittels mindestens einem Verbindungselement direkt oder indirekt mit dem Gehäuse verbunden und somit gelagert ist.

Unter "im Wesentlichen quer zu der Längsrichtung" wird insbesondere verstanden, dass nicht zwingend die Verbindung zwischen der Masse des Amplituden-Kompensators und seiner Längsrichtung einen Winkel von 90° aufweisen muss. Somit kann die Längsachse des Verbindungselementes auch einen kleineren Winkel als 90° zu der Längsrichtung der Masse und/oder des Beschleunigungsrohres aufweisen, beispielsweise kann dieser Winkel 60° betragen.

Zudem wird zusätzlich zu der üblichen Befestigung des Ultraschallwandlers an dessen Schwingungsknoten der Längsrichtung durch die Befestigung der Masse des Amplituden-Kompensators mittels mindestens eines Verbindungselementes der Ultraschallwandler stabiler mit dem Gehäuse verbunden und dadurch können größere Kräfte und Momente übertragen werden.

In einer weiteren Ausführungsform der Haltevorrichtung ist die Masse mittels mindestens drei radial gleichmäßig beabstandeten Verbindungselementen direkt oder indirekt mit dem Gehäuse verbunden.

Dadurch ist die Masse des Amplituden-Kompensators radial umlaufend mittels mindestens drei gleichmäßig verteilten Verbindungselementen an dem umgebenden Gehäuse gelagert und die Schwingungen und/oder Kräfte werden je nach Moment gleichmäßig oder ungleichmäßig über die Manteloberfläche der Masse verteilt radial in das Gehäuse abgeleitet.

Dadurch, dass das mindestens eine Verbindungselement oder die mindestens drei radial gleichmäßig beabstandeten Verbindungselemente sowie gegebenenfalls ein zusätzliches Dichtungselement, wie ein O-Ring als Dämpfungselement, in und/oder an der Masse als Ruhemasse angeordnet sind, bewegen sich diese wegen der geringen Rest-Ultraschallamplitude vernachlässigbar gering und folglich ist ein Abrieb, ein Verlust und/oder eine Erwärmung des mindestens einen Verbindungselementes, der Verbindungselemente und/oder eines Dichtungselementes sehr gering und/oder vernachlässigbar.

Um Drehmomente zu kompensieren, welche aufgrund einer Linienlagerung des Horns auftreten können, ist mittels des mindestens einen Verbindungselementes oder der Verbindungselemente jeweils eine punktuelle Verbindung direkt oder indirekt mit dem Gehäuse ausgebildet.

Durch die punktuelle Verbindung oder die punktuellen Verbindungen werden Quermomente abgefangen, durch welche ansonsten das proximale Ende des Ultraschallkonverters mit der Gehäusewand kollidieren würde, welches neben Funktionsbeeinträchtigungen zu Geräuschen, Störungen und Schäden führen kann. Des Weiteren würde ohne diese Momentübertragung mittels der punktuellen Verbindung des jeweiligen Verbindungselementes mit dem Gehäuse oder indirekt über ein anderes Bauteil mit dem Gehäuse die weiche, nachgiebige Gehäuselagerung ein wabbeliges, unpräzises und somit nachteiliges Gefühl bei dem Anwender bei der Handhabung der Haltevorrichtung und/oder Lithotripsievorrichtung verursachen. Durch die punktuelle Lagerung der Masse des Amplituden-Kompensators wird zudem eine Verstimmung des Ultraschallkonverters und ein zu großer Verlust der Schwingungsleistung in dem Gehäuse verhindert.

In einer weiteren Ausführungsform der Haltevorrichtung weist das mindestens eine Verbindungselement oder weisen die Verbindungselemente Kunststoff auf.

Dadurch, dass das mindestens eine Verbindungselement oder die Verbindungselemente Kunststoff aufweisen und somit eine Kunststoffoberfläche bei der Verbindung in Kontakt mit dem Gehäuse oder indirekt mit dem Bauteil des Gehäuses vorliegt, führen Ultraschall-Restamplituden nicht zu einem metallischen Scheppern. Folglich kann das Gehäuse oder ein Bauteil in dem Gehäuse, an dem das Verbindungselement anliegt oder die Verbindungselemente anliegen, Metall aufweisen.

Ein "Verbindungselement" ist insbesondere ein Element, welches eine mechanische Verbindung zwischen der Masse der Schwingungskompensationseinrichtung und dem Gehäuse oder einem Bauteil in dem Gehäuse herstellt. Das Verbindungselement kann insbesondere eine formschlüssige und/oder kraftschlüssige Verbindung herstellen. Ebenso kann die Masse locker verbunden und mit etwas Spiel durch das Verbindungselement oder die Verbindungselemente an und/oder in einem Bauteil im Gehäuse oder dem Gehäuse geführt sein. Bei dem Verbindungselement handelt es sich beispielsweise um einen Stift oder Bolzen. Das Verbindungselement weist bevorzugt Kunststoff auf. Um eine punktuelle Verbindung und somit eine Verbindung nur an einem Punkt oder einer kleinen Fläche des Verbindungselementes herzustellen, kann das Verbindungselement speziell geformt sein. Dazu kann das Verbindungselement beispielsweise eine Spitze oder eine Rastnase aufweisen, welche beispielsweise in eine Aussparung oder eine Riffelung in dem Gehäuse oder in einem Bauteil innerhalb des Gehäuses eingreift. Somit weist jedes Verbindungselement bevorzugt einen speziell geformten kleinflächigen Lagerpunkt aus Kunststoff auf. Durch das Kunststoffmaterial des Verbindungselementes wird gleichzeitig eine zusätzliche Dämpfung realisiert. Bei dem Verbindungselement kann es sich auch um einen Bolzen handeln, welcher Kunststoff und eine sphärische Kontaktfläche aufweist. Durch die Kugelgeometrie oder ein kugelförmiges oder halbkreisförmiges Ende des Verbindungselementes werden die Quermomente optimal auf einen Kontaktpunkt ausgerichtet und über diesen abgeleitet. Um in einfacher Weise ein Federelement herzustellen und dieses um das Beschleunigungsrohr und/oder den Hohlbolzen proximalseitig vom Horn anzuordnen, ist das mindestens eine Federelement als Rohrabschnitt ausgebildet, wobei eine Wandstärke des Rohrabschnittes kleiner als eine Materialstärke der Masse ist.

Dadurch, dass der Rohrabschnitt deutlich dünnwandiger als die Materialstärke der Masse der Schwingungskompensationseinrichtung ist, wirkt der Rohrabschnitt direkt als λ/4-Masse-Federelement. Es ist besonders vorteilhaft, dass der Amplituden-Kompensator als ein einstückiges Bauteil mit dem distalseitigen Rohrabschnitt und der proximalseitigen Masse fertigbar ist.

Die "Materialstärke" der Masse ist insbesondere die Materialdicke der Masse von ihrer Mantelfläche bis zu ihrer Innenoberfläche anliegend an dem Beschleunigungsrohr. Die "Wandstärke" ist insbesondere die Dicke des Rohrs des Rohrabschnittes.

In einer weiteren Ausführungsform der Haltevorrichtung weist die Schwingungskompensationseinrichtung einen Hohlraum und/oder eine Aussparung zum Aufnehmen eines Druckmediums und optional mindestens ein Dichtelement auf.

Dadurch wird eine weitere Funktion der Schwingungskompensationseinrichtung bereitgestellt, indem mittels der Aussparung und/oder eines Hohlraums in der Schwingungskompensationseinrichtung ein Reservoir oder eine Kammer bereitgestellt wird, in der bei einer pneumatischen Krafterzeugungseinrichtung das Druckmedium bei Bewegung des Projektils in distaler Richtung komprimiert und nach Anschlagen des Projektils an dem distalseitigen Anschlagselement das komprimierte Druckmedium zur Rückbewegung des Projektils in die entgegengesetzte, proximale Richtung verwendet werden kann. Dadurch kann eine Druckluftfeder zur Rückstellung des Projektils realisiert werden, indem mittels des komprimierten Druckmediums, insbesondere Druckluft, in der Aussparung und/oder dem Hohlraum des Schwingungskompensators ein Gegendruck aufgebaut wird, sodass bei einem abgeschalteten Pneumatikventil unter Entlüftungsbedingungen das Projektil mittels der komprimierten Druckluft wieder zuverlässig in seine Ausgangsposition zurückbewegt werden kann. Somit stellt der Amplituden-Kompensator ein Kombinationsbauteil dar, welches sowohl eine Schwingungsentkopplung als auch ein innenliegendes Volumen zur Aufnahme des komprimierten Druckmediums bereitstellt. Hierzu wird der Hohlraum und/oder die Aussparung der Schwingungskompensationseinrichtung entsprechend in ihrer Größe und somit dem Aufnahmevolumen eingestellt, um zu verhindern, dass bei einem zu kleinen Volumen der sich aufbauende Gegendruck als Druckluftfeder den Aufprall des Projektils zu stark abschwächt, sodass die Zertrümmerungsleistung abnimmt. Auf der anderen Seite kann das Volumen auch nicht beliebig vergrößert werden, da ansonsten eine zu geringe Kompression der Druckluft in dem Volumen stattfindet und das Projektil entsprechend zu wenig Rückfederdruck erfahren würde. Prinzipiell kann der Hohlraum und/oder die Aussparung frei in der Schwingungskompensationseinrichtung angeordnet sein. Bevorzugt ist der Hohlraum und/oder die Aussparung zumindest teilweise im Rohrabschnitt ausgebildet. Im Falle einer Aussparung ist diese beispielsweise in die Innenwand des Rohrabschnittes eingebracht, sodass das Volumen zur Aufnahme des Druckmediums zwischen der inneren Wandung des Rohrabschnittes an der Aussparung und der Außenoberfläche des Beschleunigungsrohres angeordnet ist. Dieses Volumen liegt insbesondere in einem Bereich von 3 ml bis 16 ml, bevorzugt von 5 ml bis 11 ml. Bei Verwendung eines Projektils mit einem Außendurchmesser von 8 mm kann das Volumen zum Aufnehmen des Druckmediums insbesondere 7,6 ml betragen. Das mittels des Hohlraums und/oder der Aussparung ausgebildete, nutzbare Volumen (Luftreservoir) für Druckluft ist insbesondere für eine hohe Projektilfrequenz und/oder Schlagfrequenz ausgelegt. Bei der Ausgestaltung des Volumens als Aussparung in beispielsweise der Innenoberfläche des Rohrabschnittes der Schwingungskompensationseinrichtung ist dieses Volumen mittels eines Dichtelementes, beispielsweise eines O-Ringes, oder mehrerer Dichtelemente, abgedichtet. Insbesondere ist das Beschleunigungsrohr und/oder die Aussparung als Druckluftkammer zum Innenraum des Gehäuses um den Ultraschallwandler abgedichtet. Bevorzugt ist dieses Luftreservoir zur Rückstellung des Projektils mit einem proximalen Dichtelement gegenüber dem Beschleunigungsrohr und mit einem distalen Dichtelement zwischen dem Verbindungsabschnitt des Amplituden-Kompensators und dem Hohlbolzenabschnitt und/oder Hohlbolzen proximalseitig des Horns abgedichtet, wodurch die Druckluft nicht in den Innenraum des Gehäuses strömen kann. Da distalseitig der Rohrabschnitt nicht zu dem Beschleunigungsrohr abgedichtet ist, kann die Druckluft durch einen Druckluftkanal zwischen der Innenoberfläche des Rohrabschnittes, des Hohlbolzenabschnittes und des Horns und der Außenoberfläche des Beschleunigungsrohres zwischen dem Luftreservoir und dem distalen Ende des Beschleunigungsrohres in proximaler Richtung oder in distaler Richtung strömen.

Das proximalseitige Dichtelement verhindert zudem ein Scheppern, da es einen metallischen Kontakt zwischen dem Beschleunigungsrohr und der unter Restamplitude stehenden Ruhemasse des Amplituden-Kompensators verhindert. Neben der Abdichtfunktion nehmen das distalseitige und proximalseitige Dichtelement des Amplituden-Kompensators zusätzlich Schwingungen auf.

Um den Bauraum innerhalb des Gehäuses optimal auszunutzen und eine effiziente Entkopplung zu erzielen, ist die Schwingungskompensationseinrichtung zumindest teilweise um das Beschleunigungsrohr angeordnet.

In einer weiteren Ausführungsform der Haltevorrichtung ist die Schwingungskompensationseinrichtung konzentrisch um das Beschleunigungsrohr angeordnet.

Dadurch wird eine gleichmäßige, radial umlaufende Entkoppelung des Beschleunigungsrohrs von der Schwingungsanregungseinrichtung erzielt.

Um eine indirekte Verbindung der Masse des Amplituden-Kompensators mit dem Gehäuse herzustellen und den vorhandenen Bauraum optimal auszunutzen, weist die Haltevorrichtung einen Platinenhalter auf, wobei der Platinenhalter zumindest teilweise um die Schwingungskompensationseinrichtung angeordnet ist und die Masse der Schwingungskompensationseinrichtung mittels des mindestens einen Verbindungselementes an dem Platinenhalter verbunden ist.

Somit weist der Platinenhalter die Doppelfunktion als Trägerelement für elektronische Bauteile innerhalb der Haltevorrichtung als auch als Lagerung für die Masse des Amplituden-Kompensators und somit als indirektes Verbindungsbauteil für die Lagerung der Masse mittels eines Verbindungselementes oder mehrerer Verbindungselemente auf.

In einer weiteren Ausführungsform weist die Haltevorrichtung distalseitig ein Horn und proximalseitig vom Horn einen Bolzen auf, wobei das Horn und der Bolzen einen distalen Abschnitt des Beschleunigungsrohrs umgeben, proximalseitig von dem Horn ein Gegenlager an dem Bolzen angeordnet ist und zwischen dem Gegenlager und dem Horn mindestens ein Piezoelement als Schwingungsanreger angeordnet und mechanisch gekoppelt ist, wobei das Horn das distalseitige Anschlagselement aufweist und/oder das Horn mit dem distalseitigen Anschlagselement und/oder Sonotrode verbindbar ist und das mindestens eine Piezoelement elektrisch mit einem zuordenbaren Ultraschallgenerator verbindbar ist, wobei die Schwingungskompensationseinrichtung proximalseitig an und/oder von dem Horn, dem Bolzen und/oder dem Gegenlager angeordnet ist.

Um den Amplituden-Kompensator distalseitig zu verbinden, weist das mindestens eine Federelement einen Verbindungsabschnitt auf, wobei der Verbindungsabschnitt einen proximalen Endabschnitt des Bolzens umgibt und/oder proximalseitig von dem Gegenlager angeordnet ist.

Dadurch kann mittels des Verbindungsabschnittes der Amplituden-Kompensator an das schwingende proximale Ende des Ultraschallwandlers und/oder des Bolzens angeschraubt werden. Folglich wird eine lösbare form- und kraftschlüssige Schraubverbindung ermöglicht.

In einer weiteren Ausführungsform der Haltevorrichtung weist die Masse einen Durchbruch und/oder an ihrer Außenoberfläche mindestens eine Aussparung in ihrer Längsrichtung zum Führen einer Leitung und/oder eines Schlauches auf.

Somit ist eine weitere Funktion des Amplituden-Kompensators realisiert, indem dieser eine Durchleitung und somit eine effiziente Ausnutzung des innerhalb des Gehäuses zur Verfügung stehenden Bauraumes ermöglicht. Dadurch wird eine Konstruktion zur Führung von Leitungen und/oder Schläuchen in Längsrichtung des Gehäuses vereinfacht, da diese an und/oder durch die Masse mit vernachlässigbar geringer Ultraschall-Restamplitude geführt und/oder abgedichtet werden. Hierbei wird durch die Aussparung oder einen Durchbruch durch die Masse in Längsrichtung ein ausreichender Raum für die Durchführung von Schläuchen und/oder elektrischen Leitungen, beispielsweise den elektrischen Leitungen von den elektrischen Kontakten der Piezoelemente zu der proximalen Kabeldurchführung und/oder der Steckbuchse am proximalen Ende des Gehäuses, bereitgestellt. Bei der Aussparung kann es sich beispielsweise um eine Ausfräsung in der Außenoberfläche und somit in der Mantelfläche der Masse durchgehend in Längsrichtung handeln. Beispielsweise können drei gleichmäßig beabstandete halbkreisförmige Ausfräsungen mit großem Radius in die Außenoberfläche der Masse eingebracht sein. Ebenso kann es sich auch um einen Durchbruch in Längsrichtung in einem äußeren Materialbereich der Masse handeln, beispielsweise um entsprechend beabstandete Langlöcher über den Querschnitt der Masse verteilt.

In einem weiteren Aspekt der Erfindung wird die Aufgabe gelöst durch eine Lithotripsievorrichtung, insbesondere intrakorporale Lithotripsievorrichtung, zum Zertrümmern von Körpersteinen, wobei die Lithotripsievorrichtung eine Sonotrode und eine Haltevorrichtung aufweist, und die Haltevorrichtung eine zuvor beschriebene Haltevorrichtung ist.

Somit wird eine Lithotripsievorrichtung mit einem Handstück bereitgestellt, welches optimal aufgrund des multifunktionalen Amplituden-Kompensators bezüglich der effizienten Ausnutzung des Bauraumes, der gezielten Handhabung durch einen Anwender, der frei anpassbaren Baulänge des Gehäuses und der unabhängigen Einstellbarkeit der ballistischen und/oder pneumatischen Stoßanregung und der Ultraschallanregung aufgrund der Schwingungsentkoppelung ausgebildet ist.

Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Im Weiteren wird die Erfindung anhand von Ausführungsbeispielen näher erläutert. Es zeigen
- Fig. 1: eine schematische dreidimensionale Darstellung einer Lithotripsievorrichtung mit einem Handstück, einem Horn und einer Sonotrode,
- Fig. 2: eine schematische dreidimensionale Darstellung des Handstückes mit einem Platinenhalter um einen Amplituden-Kompensator und ein Beschleunigungsrohr im Teilschnitt,
- Fig. 3: eine schematische dreidimensionale Darstellung des Handstückes mit dem Amplituden-Kompensator, dem Horn und dem Beschleunigungsrohr im Teilschnitt,
- Fig. 4: eine schematische Darstellung des Handstückes aus Figur 3 im Vollschnitt,
- Fig. 5: eine schematische dreidimensionale Darstellung des Amplituden-Kompensators, und
- Fig. 6: eine schematische Darstellung des Amplituden-Kompensators aus Figur 5 im Vollschnitt.

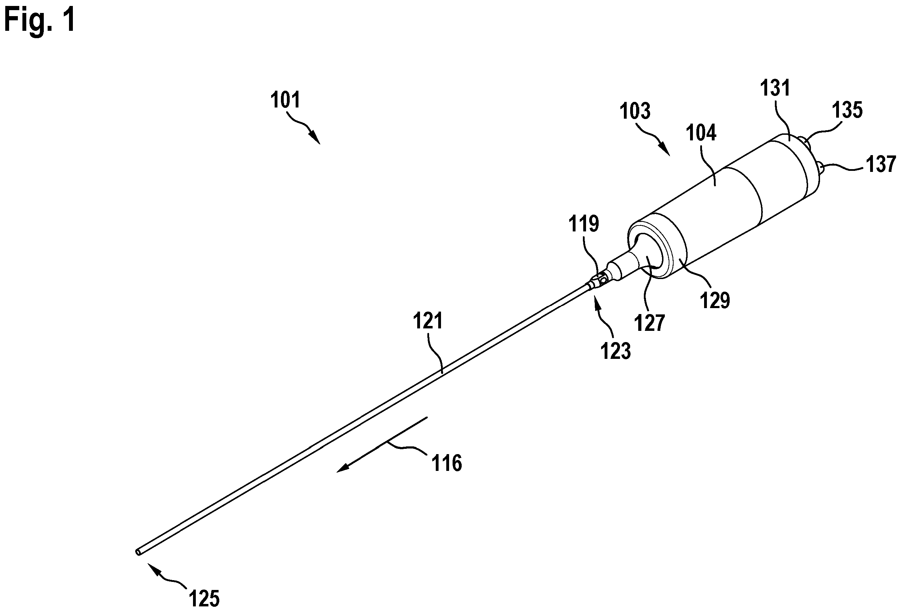

Eine Lithotripsievorrichtung 101 weist ein Handstück 103 mit einem Gehäuse 104 auf. An seinem proximalen Ende ist das Gehäuse 104 mit einem Deckel 131 abgeschlossen. An dem Deckel 131 sind proximalseitig ein elektrischer Anschluss 135 und ein Verbindungsstutzen 137 zum Zuführen von Druckluft angeordnet. Distalseitig weist das Gehäuse 104 eine Hülse 129 auf, welche ein Horn 127 umgibt. Eine Sonotrode 121 ist an ihrem proximalen Ende 123 mittels ihres Sonotrodenkopfes 119 in dem Horn 127 eingeschraubt. Ein dem proximalen Ende 123 gegenüberliegendes distales Ende 125 der Sonotrode dient zum Zertrümmern von Körpersteinen (Figur 1).

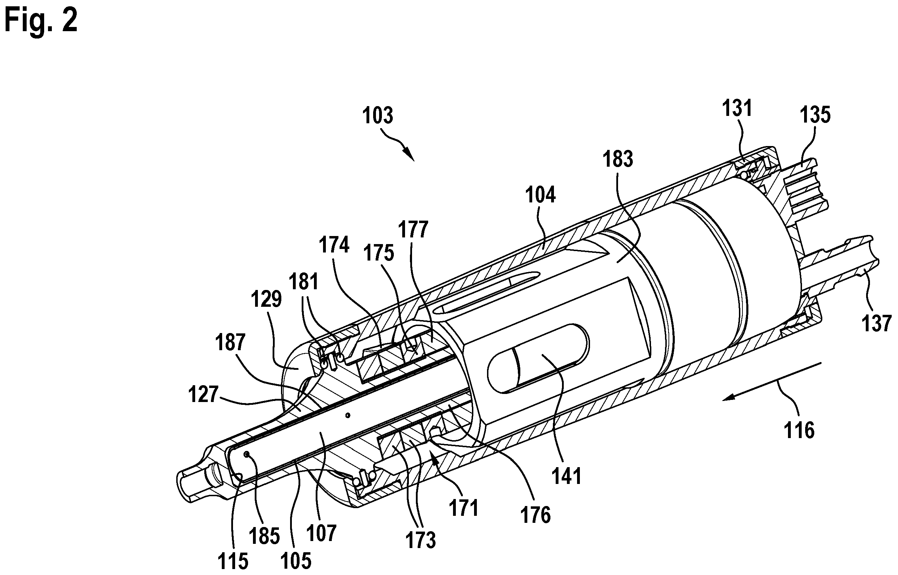

Das Horn 127 weist in einer distalen Richtung 116 einen sich verjüngenden Abschnitt auf. Proximalseitig dieses sich verjüngenden Abschnittes geht das Horn 127 einstückig in einen Hohlbolzen 176 über. Das Horn 127 ist mittels zweier O-Ringe 181 an seinem größten Querschnitt im Gehäuse 104 gelagert. Innenliegend in dem hohl ausgebildeten Horn 127 und dem sich anschließenden Hohlbolzen 176 ist ein Beschleunigungsrohr 105 angeordnet, welches sich von seinem distalen Ende 110 bis zu seinem proximalen Ende 109 entlang einer Längsmittelachse 117 erstreckt (siehe Figuren 2, 3 und 4). Das Beschleunigungsrohr 105 weist innenliegend einen Hohlraum 107 auf, in dem ein Projektil 111 beweglich angeordnet ist. Das proximale Ende 109 des Beschleunigungsrohrs 105 ist in einer Rohraufnahme 133 innerhalb des Gehäuses 104 gehalten. Der Hohlraum 107 des Beschleunigungsrohres ist fluidtechnisch mit dem Verbindungsstutzen 137 verbunden. Entlang der Längsmittelachse 117 ist das Projektil 111 in dem Hohlraum 107 des Beschleunigungsrohres 105 zwischen einem proximalseitigen Anschlagselement 113 und einem distalseitigen Anschlagselement 115 beweglich. Das distalseitige Anschlagselement 115 wird durch eine proximalseitige Wand des Horns 127 ausgebildet.

Proximalseitig des Horns 127 ist um den Hohlbolzen 176 ein Ultraschallwandler 171 angeordnet. Der Ultraschallwandler 171 weist zwei Piezoelemente 173 mit einem dazwischen angeordneten elektrischen Leiter und einem elektrischen Kontakt 174 auf. Die Piezoelemente 173 sind zwischen dem Horn 127 und einer Zwischenscheibe 175 mittels eines proximalseitigen Gegenlagers 177 eingespannt, wobei die Zwischenscheibe 175 und das Gegenlager 177 ebenfalls den Hohlbolzen 176 umgeben. Die Zwischenscheibe 175 weist an ihrer Außenoberfläche Löcher auf, in welche bei der Montage der Piezoelemente 173 auf den Hohlbolzen 176 ein Hakenschlüssel aufgesetzt wird, um bei der Montage ein Drehmoment abzuleiten und dieses von den Piezoelementen 173 fernzuhalten, da ansonsten die Gefahr besteht, dass die Piezoelemente 173 sich verdrehen und dadurch beschädigt werden.

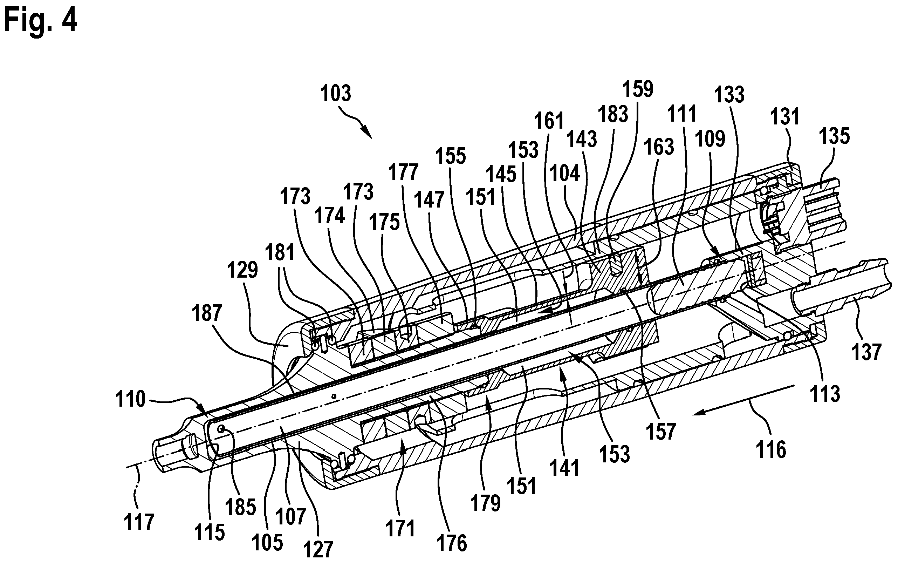

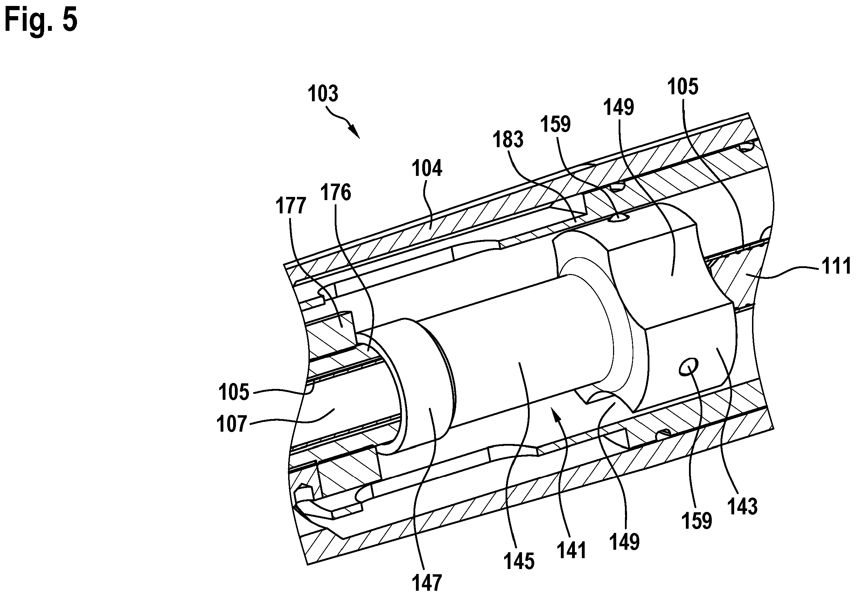

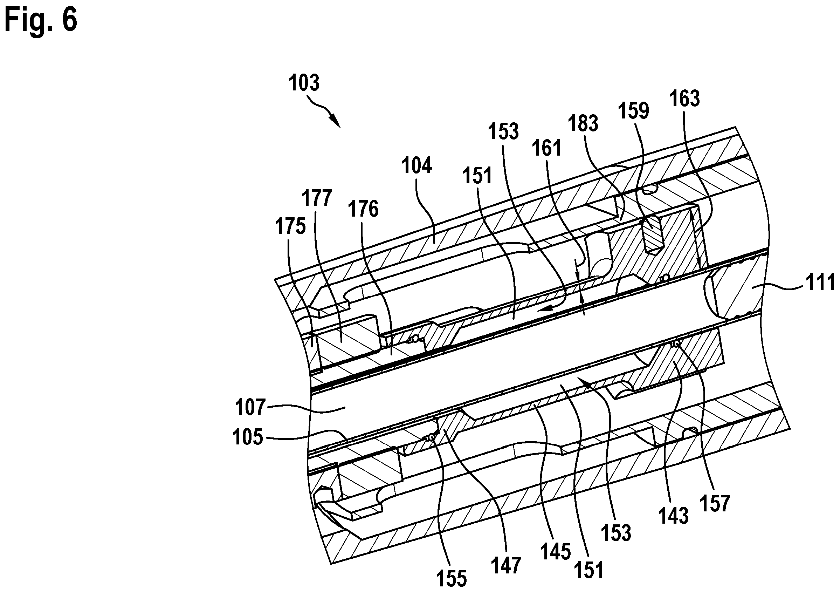

Am proximalen Ende 179 des Ultraschallwandlers 171 und im mittleren Bereich des Gehäuses 104 ist ein Amplituden-Kompensator 141 um das Beschleunigungsrohr 105 angeordnet. Der Amplituden-Kompensator 141 ist einstückig aus Aluminium gefertigt und weist proximalseitig ein Masseteil 143 und distalseitig einen Feder-Rohrabschnitt 145 auf. Der Feder-Rohrabschnitt 145 weist an seinem distalen Ende einen Verbindungsabschnitt 147 auf (Figur 5 und 6). Der Verbindungsabschnitt 147 ist auf das proximale Ende des Hohlbolzens 176 aufgeschraubt und mittels eines innenliegenden distalen O-Ringes 155 abgedichtet. Der Amplituden-Kompensator 141 weist innenliegend einen Hohlraum auf, durch den das Beschleunigungsrohr 105 geführt ist. Zusätzlich weist der Amplituden-Kompensator 141 in seiner Innenwand um den Hohlraum eine Aussparung 151 auf, welche in den Feder-Rohrabschnitt 145 und einen distalen Abschnitt des Masseteils 143 eingebracht ist, sodass der Amplituden-Kompensator 141 rundumlaufend um das Beschleunigungsrohr 105 ein Druckluftreservoir 153 aufweist (Figur 4).

Das Masseteil 143 ist mit einem proximalen O-Ring 157 an dem Beschleunigungsrohr 105 abgedichtet. Dadurch, dass der Amplituden-Kompensator 141 lediglich mit dem proximalen O-Ring 157 proximalseitig an dem Beschleunigungsrohr 105 abgedichtet ist, kann Druckluft in das durch die Aussparung 151 ausgebildete Druckluftreservoir 153 distalseitig durch einen Druckluftkanal 187 zwischen der Außenoberfläche des Beschleunigungsrohrs 105 und der Innenoberfläche des distalen Abschnittes des Amplituden-Kompensators 141, des Hohlbolzens 176 und des Horns 127 in distaler Richtung 117 aus dem Druckluftreservoir 153 austreten und durch eine Öffnung 185 am distalen Ende 110 des Beschleunigungsrohres 105 und/oder durch die offene Stirnseite am distalen Ende 110 des Beschleunigungsrohrs 105 in den Hohlraum 107 einströmen. Ebenso kann umgekehrt Druckluft aus dem Hohlraum 107 bei einer Beschleunigung des Projektils 111 in distaler Richtung 116 durch die Öffnung 185 und die offenen Stirnseite am distalen Ende 110 des Beschleunigungsrohrs 105 in den Druckluftkanal 187 als Zwischenraum zwischen der Außenoberfläche des Beschleunigungsrohrs 105 und der Innenoberfläche des Horns 127, des Hohlbolzens 176 des distalen Abschnittes des Amplituden-Kompensators 141 entgegen der distalen Richtung 116 in das Druckluftreservoir 153 gedrückt und dort gesammelt werden. Hierbei dichtet der distale O-Ring 155 zwischen dem Verbindungsabschnitt 147 des Feder-Rohrabschnittes 145 und dem proximalen Ende des Hohlbolzens 176 den Druckluftkanal 187 zum Innenraum des Gehäuses 104 ab.

Der Feder-Rohrabschnitt 145 des Amplituden-Kompensators 141 weist im Bereich der Aussparung 151 eine deutlich geringere Wandstärke 161 als eine Materialstärke 163 des Masseteils 143 zwischen der Innenoberfläche eines Platinenhalters 183 und der Außenoberfläche des Beschleunigungsrohrs 105 auf. Durch die deutlich geringere Wandstärke 161 von 1 mm im Vergleich zur Materialstärke 163 von 27 mm des Masseteils 143 weist der Feder-Rohrabschnitt 145 elastische Federeigenschaften auf.

Der Platinenhalter 183 umgibt das Beschleunigungsrohr 105 von seinem proximalen Ende 109 in distaler Richtung 116 bis einschließlich des Amplituden-Kompensators 141 und des Gegenhalters 177. Das Masseteil 143 des Amplituden-Kompensators 141 ist an seiner Mantelfläche mittels drei radial gleichmäßig beabstandeter Kunststoff-Stifte 159 punktuell an der Innenoberfläche des Platinenhalters 183 geführt oder kraft- und formschlüssig befestigt. Der Platinenhalter 183 steht wiederum radial umlaufend mit der Innenseite des Gehäuses 104 in Kontakt, sodass der Amplituden-Kompensator 141 indirekt über den Platinenhalter 183 mit dem Gehäuse 104 in radialer Richtung verbunden ist. Dadurch ist das proximale Ende des Masseteils 143 gerade frei von einer Verbindung zu dem Gehäuse 104 und dem Deckel 131 in proximaler Richtung.

Des Weiteren weist das Masseteil 143 drei teilkreisförmige, in distaler Richtung 116 durchgehende Durchleitungsaussparungen 149 zum Durchführen von in den Figuren nicht gezeigten elektrischen Leitungen von dem elektrischen Anschluss 135 zu dem Ultraschallwandler 171 auf.

Mit der kombinierten Lithotripsievorrichtung 101 mit einer Schwingungsanregung der Sonotrode 121 mittels des Ultraschallwandlers 171 und einem pneumatischen Antrieb zur Stoßanregung der Sonotrode 121 mittels des Projektils 111 werden folgende Arbeitsgänge durchgeführt.

Mittels eines nicht in den Figuren gezeigten Ultraschallgenerators wird der Ultraschallwandler 171 mit einer Spannung an dem elektrischen Kontakt 174 beaufschlagt, wodurch eine Verformung der Piezoelemente 173 innerhalb des Ultraschallwandlers 171 erfolgt und dadurch eine Ultraschallschwingung indiziert wird. Die erzeugte Ultraschallschwingung wird aufgrund des konischen Abschnittes des Horns 127 in die Sonotrode 121 eingeleitet, wodurch die Sonotrode 121 zu einer Schwingungswelle in einer longitudinalen Schwingung als auch in Querrichtung angeregt wird.

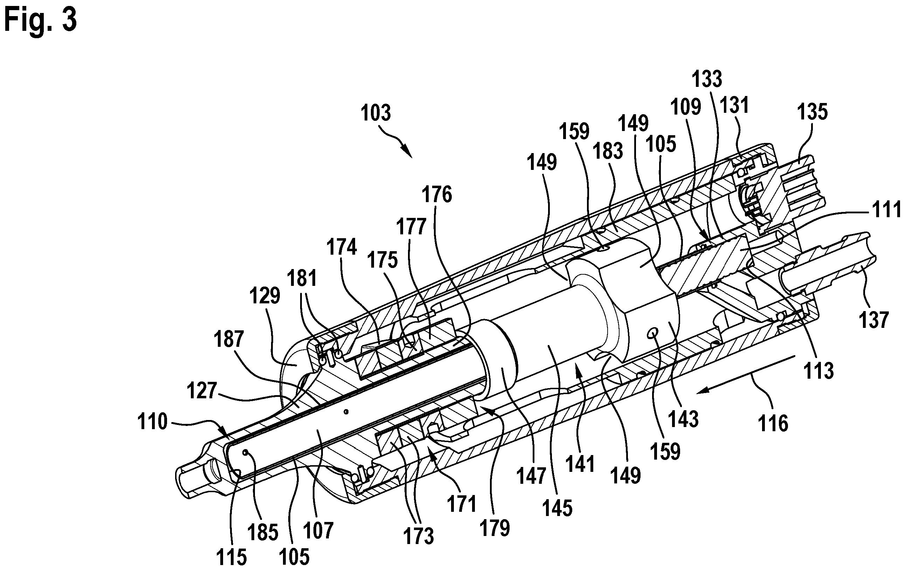

Gleichzeitig wird mittels einer nicht gezeigten Krafterzeugungseinrichtung Druckluft durch den Verbindungsstutzen 137 in den Hohlraum 107 am proximalen Ende 109 des Beschleunigungsrohrs 105 gedrückt, wodurch sich das Projektil 111 vom proximalen Ende 109 als Ausgangszustand (siehe Figur 3 und 4) in distaler Richtung 116 durch den Hohlraum 107 entlang der Längsmittelachse 117 vom proximalseitigen Anschlagselement 113 zum distalseitigen Anschlagselement 115 bewegt und durch Aufschlagen auf das distalseitige Anschlagselement 115 über das distale Ende des Horns 127 und den Sonotrodenkopf 119 der Schlag der Projektils 111 auf die Sonotrode 121 übertragen wird. Durch die Beschleunigung des Projektils 111 in distaler Richtung 116 wird die Luft im distalen Abschnitt des Hohlraumes 107 innerhalb des Beschleunigungsrohres 105 komprimiert und entweicht durch die Öffnung 185 und den Druckluftkanal 187 zwischen der Außenoberfläche des Beschleunigungsrohrs 105 und der Innenoberfläche des Hornes 127, des Hohlbolzens 176 und des distalen Abschnittes des Amplituden-Kompensators 141 entgegen der distalen Richtung 116 in das Druckluftreservoir 153 des Amplituden-Kompensators 141, wobei die Druckluft in dem Druckluftreservoir 153 komprimiert wird. Durch Aufprall des Projektils 111 gegen das distalseitige Anschlagselement 115 wird das Projektil 111 repulsiert und durch gleichzeitiges Schließen der zuströmenden Druckluft und Entlüftung durch den Verbindungsstutzen 137 strömt nun die in dem Druckluftreservoir 153 komprimierte Druckluft in distaler Richtung 116 durch den Druckluftkanal 187, die Öffnung 185 und die offene distale Stirnseite des Beschleunigungsrohrs 105 in den Hohlraum 107 und drückt das Projektil 111 wieder zum proximalen Ende 109 des Beschleunigungsrohres 105 zurück, bis der Ausgangszustand (Figur 3 und 4) wieder erreicht ist. Diese Schlaganregung der Sonotrode 121 durch Auftreffen des Projektils 111 auf das distalseitige Anschlagselement 115 wird regelmäßig wiederholt.

Die mittels des Ultraschallwandlers 171 erzeugten Ultraschallschwingungen weisen eine Frequenz von circa 27 kHz auf, auf welche der Amplituden-Kompensator 141 exakt abgestimmt ist. Dadurch, dass der angekoppelte Amplituden-Kompensator 141 eine λ/4-Geometrie aufweist, welche der Resonanzfrequenz des Ultraschallwandlers 171 entspricht, klingt die Amplitude der mittels des Ultraschallwandlers 171 erzeugten Schwingungswelle nach proximal entlang des Feder-Rohrabschnitts 145 kontinuierlich auf quasi null im proximalen Massenteil 143 ab, und der Ultraschallwandler 171 wird durch den Amplituden-Kompensator 141 nicht oder kaum verstimmt. Dabei bewegt sich das Masseteil 143 als Ruhemasse aufgrund der geringen Rest-Ultraschallamplitude, wenn überhaupt, nur vernachlässigbar gering. Hierbei wirken die radial umlaufend angeordneten Kunststoff-Stifte 159 zur punktuellen Lagerung und der proximale O-Ring 157 zusätzlich dämpfend, sodass ein Abrieb, anderweitige Beschädigung und eine Erwärmung im Masseteil 143 vernachlässigbar sind. Durch die punktuelle Lagerung mittels der Kunststoff-Stifte 159, über welche etwaig vorhandene Quermomente radial nach außen abgegeben werden, wird zudem ein metallisches Scheppern an dem Platinenhalter 183 verhindert.

Neben den Kunststoff-Stiften 159 wird auch durch den proximalen O-Ring 157 des Amplituden-Kompensators 141 ein Scheppern verhindert, da dadurch ein metallischer Kontakt zwischen dem Beschleunigungsrohr 105 und dem unter Restamplitude stehenden Masseteil 143 als Ruhemasse verhindert wird.

Dadurch, dass der Amplituden-Kompensator 141 mittels der Kunststoff-Stifte 159 an seiner Mantelfläche radial nach außen an dem Platinenhalter 183 gelagert ist und das proximale Ende des Masseteils 143 frei in proximaler Richtung und gerade nicht mit dem Gehäuse 104 und dem Deckel 131 verbunden ist, hat das Beschleunigungsrohr 105 eine optimal auf die Schlagwirkung abgestimmte Länge, sodass der pneumatische Antrieb des Projektils 111 im Beschleunigungsrohr 105 unabhängig von der erzeugten Ultraschallschwingung mittels des Ultraschallwandlers 171 betreibbar ist und beide Antriebe unabhängig voneinander einstellbar sind.

Somit sind bei Verwendung der Sonotrode 121 zur direkten Zertrümmerung von Körpersteinen sowohl die Schwingungsanregung der Sonotrode 121 mittels des Ultraschallwandlers 171 als auch die Schlaganregung des Projektils 111 mit einer effektiven hohen Zertrümmerungsleistung nutzbar.

Zudem kompensiert der Amplituden-Kompensator 141 durch die punktuelle Lagerung mittels der Kunststoff-Stifte 159 radial nach außen an dem Platinenhalter 183 und darüber an dem Gehäuse 104 Drehmomente, welche aufgrund einer nachgiebigen Linienlagerung des Horns 127 mittels der zwei O-Ringe 181 auftreten können. Durch dieses Abfangen von möglichen Quermomenten wird verhindert, dass das proximale Ende des Ultraschallwandlers 171 mit der Innenwand des Gehäuses 104 kollidiert und folglich werden entsprechende Geräusche, Störungen und/oder Schäden verhindert. Durch die punktuelle Lagerung mittels der Kunststoff-Stifte 159 wird zudem dem Anwender der kombinierten Lithotripsievorrichtung 101 eine präzise Handhabung des Gehäuses 104 und somit eine präzise Führung der gesamten Lithotripsievorrichtung 101 ermöglicht.

Dadurch, dass der Amplituden-Kompensator 141 zudem das Druckluftreservoir 153 bereitstellt, ist gleichzeitig die Funktion der Rückstellung des Projektils 111 in dem Amplituden-Kompensator 141 integriert, wodurch eine rasche Rückführung des Projektils 111 und somit eine hohe Schlagfrequenz bei geringem Bauraum ermöglicht ist. Vor allem ist dadurch ein zusätzlicher, gegenüber dem Projektil 111 distalseitiger Drucklufteinlass und eine entsprechende Ventilumschaltung zur Projektilrückführung, welche aufwändig und großbauend sind, nicht notwendig.

Somit wird eine kombinierte Lithotripsievorrichtung 101 mit einem multifunktionalen Amplituden-Kompensator 141 bereitgestellt, welcher das Beschleunigungsrohr 105 von der starken Ultraschallschwingung des Ultraschallwandlers 171 entkoppelt, ein Druckluftreservoir 153 zur Rückstellung des Projektils 111 bereitstellt, Quermomente abfängt und diese gezielt radial nach außen durch eine punktuelle Lagerung mittels Kunststoff-Stiften 159 ableitet, wodurch eine proximalseitige Länge des Gehäuses 104 frei und unabhängig auslegbar ist.

Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen. Die Erfindung betrifft eine Haltevorrichtung für eine Lithotripsievorrichtung zum Zertrümmern von Körpersteinen, wobei die Haltevorrichtung ein Gehäuse mit einem distalen Ende und einem proximalen Ende aufweist und an dem distalen Ende eine Sonotrode verbindbar ist, wobei in dem Gehäuse ein Beschleunigungsrohr mit einer Längsmittelachse, einem Hohlraum, einem proximalen Ende, einem distalen Ende und mit einem bewegbaren Projektil innerhalb des Hohlraums zur Stoßanregung der Sonotrode, ein proximalseitiges Anschlagselement am proximalen Ende und ein distalseitiges Anschlagselement am distalen Ende des Beschleunigungsrohres angeordnet sind, und der Haltevorrichtung eine Krafterzeugungseinrichtung zum Erzeugen einer Kraft zum Hin- und/oder Zurückbewegen des Projektils zwischen dem proximalseitigen Anschlagselement und dem distalseitigen Anschlagselement zuordenbar ist, und in dem Gehäuse eine Schwingungsanregungseinrichtung zur Schwingungsanregung der Sonotrode angeordnet ist, wobei die Haltevorrichtung eine Schwingungskompensationseinrichtung mit mindestens einer Masse und mindestens einem Federelement aufweist, sodass mittels der Schwingungskompensationseinrichtung das Beschleunigungsrohr von der Schwingungsanregung mittels der Schwingungsanregungseinrichtung entkoppelbar ist. Des Weiteren betrifft die Erfindung eine Lithotripsievorrichtung zum Zertrümmern von Körpersteinen.

### Bezugszeichenliste

- 101: Lithotripsievorrichtung
- 103: Handstück
- 104: Gehäuse
- 105: Beschleunigungsrohr
- 107: Hohlraum
- 109: proximales Ende
- 110: distales Ende
- 111: Projektil
- 113: proximalseitiges Anschlagselement
- 115: distalseitiges Anschlagselement
- 116: distale Richtung
- 117: Längsmittelachse
- 119: Sonotrodenkopf
- 121: Sonotrode
- 123: proximales Ende der Sonotrode
- 125: distales Ende der Sonotrode
- 127: Horn
- 129: Hülse
- 131: Deckel
- 133: Rohraufnahme
- 135: elektrischer Anschluss
- 137: Verbindungsstutzen
- 141: Amplituden-Kompensator
- 143: Masseteil
- 145: Feder-Rohrabschnitt
- 147: Verbindungsabschnitt
- 149: Durchleitungsaussparung
- 151: Aussparung
- 153: Druckluftreservoir
- 155: Distaler O-Ring
- 157: Proximaler O-Ring
- 159: Kunststoff-Stift
- 161: Wandstärke
- 163: Materialstärke
- 171: Ultraschallwandler
- 173: Piezoelement
- 174: elektrischer Kontakt
- 175: Zwischenscheibe
- 176: Hohlbolzen
- 177: Gegenlager
- 179: Proximales Ende des Ultraschallwandlers
- 181: O-Ring
- 183: Platinenhalter
- 185: Öffnung
- 187: Druckluftkanal

## Patentansprüche

1. Haltevorrichtung (103) für eine Lithotripsievorrichtung (101) zum Zertrümmern von Körpersteinen, wobei die Haltevorrichtung (103) ein Gehäuse (104) mit einem distalen Ende und einem proximalen Ende aufweist und an dem distalen Ende eine Sonotrode (121) verbindbar ist, wobei in dem Gehäuse (104) ein Beschleunigungsrohr (105) mit einer Längsmittelachse (117), einem Hohlraum (107), einem proximalen Ende (109), einem distalen Ende (110) und mit einem bewegbaren Projektil (111) innerhalb des Hohlraums (107) zur Stoßanregung der Sonotrode (121), ein proximalseitiges Anschlagselement (113) am proximalen Ende (109) und ein distalseitiges Anschlagselement (115) am distalen Ende (110) des Beschleunigungsrohres (105) angeordnet sind, und der Haltevorrichtung (103) eine Krafterzeugungseinrichtung zum Erzeugen einer Kraft zum Hin- und/oder Zurückbewegen des Projektils (111) zwischen dem proximalseitigen Anschlagselement (113) und dem distalseitigen Anschlagselement (115) zuordenbar ist, und in dem Gehäuse (104) eine Schwingungsanregungseinrichtung (171) zur Schwingungsanregung der Sonotrode (121) angeordnet ist, **dadurch gekennzeichnet, dass** die Haltevorrichtung (103) eine Schwingungskompensationseinrichtung (141) mit mindestens einer Masse (143) und mindestens einem Federelement (145) aufweist, sodass mittels der Schwingungskompensationseinrichtung (141) das Beschleunigungsrohr (105) von der Schwingungsanregung mittels der Schwingungsanregungseinrichtung (171) entkoppelbar ist.

2. Haltevorrichtung (103) nach Anspruch 1, **dadurch gekennzeichnet, dass** die Masse (143) in ihrer Längsrichtung und/oder an ihrem proximalen Ende frei von einer Verbindung mit dem Gehäuse (104) angeordnet ist.

3. Haltevorrichtung (103) nach Anspruch 1 oder 2, **dadurch gekennzeichnet, dass** die Masse (143) im Wesentlichen quer zu ihrer Längsrichtung mittels mindestens eines Verbindungselementes (159) direkt oder indirekt mit dem Gehäuse (104) verbunden ist.

4. Haltevorrichtung (103) nach Anspruch 3, **dadurch gekennzeichnet, dass** die Masse (143) mittels mindestens drei radial gleichmäßig beabstandeten Verbindungselementen (159) direkt oder indirekt mit dem Gehäuse (104) verbunden ist.

5. Haltevorrichtung (103) nach einem der Ansprüche 3 oder 4, **dadurch gekennzeichnet, dass** mittels des mindestens einen Verbindungselementes oder der Verbindungselemente (159) jeweils eine punktuelle Verbindung direkt oder indirekt mit dem Gehäuse (104) ausgebildet ist.

6. Haltevorrichtung (103) nach einem der Ansprüche 3 bis 5, **dadurch gekennzeichnet, dass** das mindestens eine Verbindungselement oder die Verbindungselemente (159) Kunststoff aufweist oder aufweisen.

7. Haltevorrichtung (103) nach einem der vorherigen Ansprüche, **dadurch gekennzeichnet, dass** das mindestens eine Federelement als Rohrabschnitt (145) ausgebildet ist, wobei eine Wandstärke (161) des Rohrabschnittes (145) kleiner als eine Materialstärke (163) der Masse (143) ist.

8. Haltevorrichtung (103) nach einem der vorherigen Ansprüche, **dadurch gekennzeichnet, dass** die Schwingungskompensationseinrichtung (141) einen Hohlraum und/oder eine Aussparung (151) zum Aufnehmen eines Druckmediums und optional mindestens ein Dichtelement (155, 157) aufweist.

9. Haltevorrichtung (103) nach einem der vorherigen Ansprüche, **dadurch gekennzeichnet, dass** die Schwingungskompensationseinrichtung (141) zumindest teilweise um das Beschleunigungsrohr (105) angeordnet ist.

10. Haltevorrichtung (103) nach einem der vorherigen Ansprüche, **dadurch gekennzeichnet, dass** die Schwingungskompensationseinrichtung (141) konzentrisch um das Beschleunigungsrohr (105) angeordnet ist.

11. Haltevorrichtung (103) nach einem der vorherigen Ansprüche, **dadurch gekennzeichnet, dass** die Haltevorrichtung (103) einen Platinenhalter (183) aufweist, wobei der Platinenhalter (183) zumindest teilweise um die Schwingungskompensationseinrichtung (141) angeordnet ist und die Masse (143) der Schwingungskompensationseinrichtung (141) mittels des mindestens einem Verbindungselementes (159) an dem Platinenhalter (183) verbunden ist.

12. Haltevorrichtung (103) nach einem der vorherigen Ansprüche, **dadurch gekennzeichnet, dass** die Haltevorrichtung (103) distalseitig ein Horn (127) und proximalseitig vom Horn (127) einen Bolzen (176) aufweist, wobei das Horn (127) und der Bolzen (176) einen distalen Abschnitt des Beschleunigungsrohrs (105) umgeben, proximalseitig von dem Horn (127) ein Gegenlager (177) an dem Bolzen (176) angeordnet ist und zwischen dem Gegenlager (177) und dem Horn (127) mindestens ein Piezoelement (173) als Schwingungsanreger angeordnet und mechanisch gekoppelt ist, wobei das Horn (127) das distalseitige Anschlagselement (115) aufweist und/oder das Horn (127) mit dem distalseitigen Anschlagselement (115) und/oder der Sonotrode (121) verbindbar ist und das mindestens eine Piezoelement (173) elektrisch mit einem zuordenbaren Ultraschallgenerator verbindbar ist, wobei die Schwingungskompensationseinrichtung (141) proximalseitig an und/oder von dem Horn (127), dem Bolzen (176) und/oder dem Gegenlager (177) angeordnet ist.

13. Haltevorrichtung nach Anspruch 12, **dadurch gekennzeichnet, dass** das mindestens eine Federelement (145) einen Verbindungsabschnitt (147) aufweist, wobei der Verbindungsabschnitt (147) einen proximalen Endabschnitt des Bolzens (176) umgibt und/oder proximalseitig von dem Gegenlager (177) angeordnet ist.

14. Haltevorrichtung (103) nach einem der vorherigen Ansprüche, **dadurch gekennzeichnet, dass** die Masse (143) einen Durchbruch und/oder an ihrer Außenoberfläche mindestens eine Aussparung (149) in ihrer Längsrichtung zum Führen einer Leitung und/oder eines Schlauches aufweist.

15. Lithotripsievorrichtung (101), insbesondere intrakorporale Lithotripsievorrichtung, zum Zertrümmern von Körpersteinen, wobei die Lithotripsievorrichtung (101) eine Sonotrode (121) und eine Haltevorrichtung (103) aufweist, **dadurch gekennzeichnet, dass** die Haltevorrichtung eine Haltevorrichtung (103) nach einem der Ansprüche 1 bis 14 ist.

## Claims

1. A holding device (103) for a lithotripsy device (101) for fragmenting body stones, wherein the holding device (103) has a housing (104) with a distal end and a proximal end, and a sonotrode (121) can be connected to the distal end, wherein in the housing (104) are arranged an acceleration tube (105) with a longitudinal centre axis (117), a cavity (107), a proximal end (109), a distal end (110) and with a movable projectile (111) within the cavity (107) for impact excitation of the sonotrode (121), a proximal-side stop element (113) at the proximal end (109) and a distal-side stop element (115) at the distal end (110) of the acceleration tube (105), and a force generation apparatus for generating a force for moving the projectile (111) back and forth between the proximal-side stop element (113) and the distal-side stop element (115) can be assigned to the holding device (103), and in the housing (104) is arranged an oscillation excitation apparatus (171) for oscillation excitation of the sonotrode (121), **characterised in that** the holding device (103) has a vibration compensation apparatus (141) with at least one mass (143) and at least one spring element (145), such that by means of the vibration compensation apparatus (141) the acceleration tube (105) can be decoupled from the excitation of vibrations by means of the vibration excitation apparatus (171).

2. The holding device (103) according to claim 1, **characterised in that** the mass (143) is arranged without a connection to the housing (104) in its longitudinal direction and/or at its proximal end.

3. The holding device (103) according to claim 1 or 2, **characterised in that** the mass (143) is connected directly or indirectly to the housing (104) substantially transversely to its longitudinal direction by means of at least one connecting element (159).

4. The holding device (103) according to claim 3, **characterised in that** the mass (143) is connected directly or indirectly to the housing (104) by means of at least three radially uniformly spaced connecting elements (159).

5. The holding device (103) according to one of claims 3 or 4, **characterised in that** a punctiform connection is formed directly or indirectly with the housing (104) by means of the at least one connecting element or the connecting elements (159).

6. The holding device (103) according to one of claims 3 to 5, **characterised in that** the at least one connecting element or the connecting elements (159) comprises or comprise plastic.

7. The holding device (103) according to one of the preceding claims, **characterised in that** the at least one spring element is designed as a tube section (145), wherein a wall thickness (161) of the tube section (145) is smaller than a material thickness (163) of the mass (143).

8. The holding device (103) according to one of the preceding claims, **characterised in that** the vibration compensation apparatus (141) has a cavity and/or a recess (151) for receiving a pressure medium and optionally at least one sealing element (155, 157).

9. The holding device (103) according to one of the preceding claims, **characterised in that** the vibration compensation apparatus (141) is arranged at least partially around the acceleration tube (105).

10. The holding device (103) according to one of the preceding claims, **characterised in that** the vibration compensation apparatus (141) is arranged concentrically around the acceleration tube (105).

11. The holding device (103) according to one of the preceding claims, **characterised in that** the holding device (103) comprises a circuit board holder (183), wherein the circuit board holder (183) is arranged at least partially around the vibration compensation apparatus (141), and the mass (143) of the vibration compensation apparatus (141) is connected to the circuit board holder (183) by means of the at least one connecting element (159).

12. The holding device (103) according to one of the preceding claims, **characterised in that** the holding device (103) has a horn (127) on the distal side and a bolt (176) on the proximal side of the horn (127), wherein the horn (127) and the bolt (176) surround a distal section of the acceleration tube (105), a counter bearing (177) is arranged on the bolt (176) on the proximal side of the horn (127) and at least one piezo element (173) is arranged and mechanically coupled between the counter bearing (177) and the horn (127) as a vibration exciter, wherein the horn (127) has the distal-side stop element (115) and/or the horn (127) can be connected to the distal-side stop element (115) and/or the sonotrode (121), and the at least one piezo element (173) can be electrically connected to an assignable ultrasound generator, wherein the vibration compensation apparatus (141) is arranged on the proximal side on and/or of the horn (127), the bolt (176) and/or the counter bearing (177).

13. The holding device according to claim 12, **characterised in that** the at least one spring element (145) has a connecting section (147), wherein the connecting section (147) surrounds a proximal end section of the bolt (176) and/or is arranged on the proximal side of the counter bearing (177).

14. The holding device (103) according to one of the preceding claims, **characterised in that** the mass (143) has a cut-out and/or at least one recess (149) on its outer surface in its longitudinal direction for guiding a line and/or a hose.

15. A lithotripsy device (101), in particular intracorporeal lithotripsy device, for fragmenting body stones, wherein the lithotripsy device (101) comprises a sonotrode (121) and a holding device (103), **characterised in that** the holding device is a holding device (103) according to one of claims 1 to 14.

## Revendications

1. Dispositif de maintien (103) pour un dispositif de lithotripsie (101) pour fragmenter des calculs corporels, dans lequel le dispositif de maintien (103) a un boîtier (104) avec une extrémité distale et une extrémité proximale, et une sonotrode (121) peut être reliée à l'extrémité distale, dans lequel, agencé dans le boîtier (104), il y a un tube d'accélération (105) avec un axe central longitudinal (117), une cavité (107), une extrémité proximale (109), une extrémité distale (110) et avec un projectile mobile (111) à l'intérieur de la cavité (107) pour l'excitation par impact de la sonotrode (121), un élément d'arrêt de côté proximal (113) au niveau de l'extrémité proximale (109) et un élément d'arrêt de côté distal (115) au niveau de l'extrémité distale (110) du tube d'accélération (105), et un appareil de génération de force pour générer une force pour déplacer le projectile (111) d'avant en arrière entre l'élément d'arrêt de côté proximal (113) et l'élément d'arrêt de côté distal (115) peut être assigné au dispositif de maintien (103), et dans le boîtier (104) est agencé un appareil d'excitation d'oscillation (171) pour l'excitation d'oscillation de la sonotrode (121), **caractérisé en ce que** le dispositif de maintien (103) a un appareil de compensation des vibrations (141) avec au moins une masse (143) et au moins un élément de ressort (145), de telle sorte que l'appareil de compensation des vibrations (141) permette de découpler le tube d'accélération (105) de l'excitation des vibrations au moyen de l'appareil d'excitation des vibrations (171).

2. Dispositif de maintien (103) selon la revendication 1, **caractérisé en ce que** la masse (143) est agencée sans une liaison au boîtier (104) dans sa direction longitudinale et/ou à son extrémité proximale.

3. Dispositif de maintien (103) selon la revendication 1 ou 2, **caractérisé en ce que** la masse (143) est reliée directement ou indirectement au boîtier (104) sensiblement transversalement à sa direction longitudinale au moyen d'au moins un élément de liaison (159).

4. Dispositif de maintien (103) selon la revendication 3, **caractérisé en ce que** la masse (143) est reliée directement ou indirectement au boîtier (104) au moyen d'au moins trois éléments de liaison (159) uniformément espacés de manière radial.

5. Dispositif de maintien (103) selon l'une des revendications 3 ou 4, **caractérisé en ce qu'**une liaison punctiforme est formée directement ou indirectement avec le boîtier (104) au moyen de l'au moins un élément de liaison ou des éléments de liaison (159).

6. Dispositif de maintien (103) selon l'une des revendications 3 à 5, **caractérisé en ce que** l'au moins un élément de liaison ou les éléments de liaison (159) comprend ou comprennent du plastique.

7. Dispositif de maintien (103) selon l'une des revendications précédentes, **caractérisé en ce que** l'au moins un élément de ressort est conçu comme une section de tube (145), dans lequel l'épaisseur de paroi (161) de la section de tube (145) est inférieure à une épaisseur de matériau (163) de la masse (143).

8. Dispositif de maintien (103) selon l'une des revendications précédentes, **caractérisé en ce que** l'appareil de compensation des vibrations (141) a une cavité et/ou un renfoncement (151) pour recevoir un agent de pression et éventuellement au moins un élément d'étanchéité (155, 157).

9. Dispositif de maintien (103) selon l'une des revendications précédentes, **caractérisé en ce que** l'appareil de compensation des vibrations (141) est agencé au moins partiellement autour du tube d'accélération (105).

10. Dispositif de maintien (103) selon l'une des revendications précédentes, **caractérisé en ce que** l'appareil de compensation des vibrations (141) est agencé concentriquement autour du tube d'accélération (105).

11. Dispositif de maintien (103) selon l'une des revendications précédentes, **caractérisé en ce que** le dispositif de maintien (103) comprend un support de carte de circuit imprimé (183), dans lequel le support de carte de circuit imprimé (183) est agencé au moins partiellement autour de l'appareil de compensation des vibrations (141), et la masse (143) de l'appareil de compensation des vibrations (141) est reliée au support de carte de circuit imprimé (183) au moyen d'au moins un élément de liaison (159).

12. Dispositif de maintien (103) selon l'une des revendications précédentes, **caractérisé en ce que** le dispositif de maintien (103) a une corne (127) sur le côté distal et un boulon (176) sur le côté proximal de la corne (127), dans lequel la corne (127) et le boulon (176) entourent une section distale du tube d'accélération (105), un contre-palier (177) est agencé sur le boulon (176) sur le côté proximal de la corne (127) et au moins un élément piézoélectrique (173) est agencé et couplé de manière mécanique entre le contre-palier (177) et la corne (127) en tant qu'un excitateur de vibrations, dans lequel la corne (127) a l'élément d'arrêt de côté distal (115) et/ou la corne (127) peut être reliée à l'élément d'arrêt de côté distal (115) et/ou à la sonotrode (121), et l'au moins un élément piézoélectrique (173) peut être relié électriquement à un générateur d'ultrasons qui peut être assigné, dans lequel l'appareil de compensation des vibrations (141) est agencé sur le côté proximal sur et/ou de la corne (127), le boulon (176) et/ou le contre-palier (177).

13. Dispositif de maintien selon la revendication 12, **caractérisé en ce que** l'au moins un élément de ressort (145) a une section de liaison (147), dans lequel la section de liaison (147) entoure une section d'extrémité proximale du boulon (176) et/ou est agencée sur le côté proximal du contre-palier (177).

14. Dispositif de maintien (103) selon l'une des revendications précédentes, **caractérisée en ce que** la masse (143) a une découpe et/ou au moins un renfoncement (149) sur sa surface extérieure dans sa direction longitudinale pour guider une ligne et/ou un tuyau.

15. Dispositif de lithotripsie (101), en particulier un dispositif de lithotripsie intracorporelle, pour fragmenter des calculs corporels, dans lequel le dispositif de lithotritie (101) comprend une sonotrode (121) et un dispositif de maintien (103), **caractérisé en ce que** le dispositif de maintien est un dispositif de maintien (103) selon l'une des revendications 1 à 14.
